# EUROPEAN PATENT SPECIFICATION

(11) **EP 2 303 147 B1**
(45) Date of publication and mention of the grant of the patent: **27.08.2014**
(21) Application number: 09785342.8
(22) Date of filing: 16.07.2009
(51) Int. Cl.: A61B 17/16

(54) **AN INSTRUMENT FOR FORMING A CAVITY WITHIN A BONE**
INSTRUMENT ZUR BILDUNG EINER KAVITÄT IN EINEM KNOCHEN
INSTRUMENT DESTINÉ À FORMER UNE CAVITÉ À L'INTÉRIEUR D'UN OS

(30) Priority: 29.07.2008 GB 0813818
(43) Date of publication of application: 06.04.2011
(73) Proprietor: DePuy International Limited, Leeds LS11 8DT (GB)
(72) Inventor: GREEN, Ivan, Yorkshire LS11 8DT (GB); ANDERSON, James, Yorkshire LS11 8DT (GB); RANGAIAH, Chetan, Yorkshire LS11 8DT (GB)
(74) Representative: Burton, Nick
(86) International application number: PCT/GB2009/050866
(87) International publication number: WO 2010/013027

(56) References cited:
- EP-A- 0 008 622
- EP-A- 0 370 204
- WO-A-01/74258
- DE-C- 931 024
- FR-A- 2 479 051
- US-A1- 2001 034 526
- US-A1- 2003 045 885
- US-A1- 2005 240 193

## Description

The present invention relates to a surgical instrument for forming a cavity within a bone. The present invention can be used in a method of forming a cavity within a bone. In particular, the method is suitable for treating avascular necrosis.

It can be desirable to form a cavity within a bone during treatment of a bone defect. This can arise for example in a patient suffering from avascular necrosis (AVN), which is also known as osteonecrosis (ON), ischemic bone necrosis, or aseptic necrosis. AVN results from the temporary or permanent loss of circulation to the bone tissue, and gives rise to localized death of the bone tissue. The loss of proper blood flow can result from trauma, or compromising conditions such as prolonged steroid use, alcohol use, gout diabetes, pancreatitis, venous occlusion, decompression disease, radiation therapy, chemotherapy, and Gaucher's disease.

The condition can give rise to severe pain and limitation of movement within a short period, with a 70 to 80% chance of complete collapse of the bone and surrounding articulating surface after three years if left untreated. Joint replacement can be necessary for many patients.

Treatments for AVN which focus on salvaging the head of the femur or other bone or joint include core decompression, osteotomy, bone grafting, and vascularized fibular grafting.

US-6679890 discloses a method and device for treating AVN of the femoral head. The device disclosed in US-6679890 augments the femoral head with bone cement. An open ended and fenestrated tube is inserted through a hole into the femoral neck and uncured bone cement is injected and cured at high pressure. The cavity which is formed in the femur, extending through the neck into the head, has a constant cross-section, such as might be formed using a rotating drill or reamer or burr instrument.

Frequently, the portion of a bone which is affected by AVN is concentrated in the superior anterior region of the femoral head. The affected portion can have a generally flat shape when the bone is viewed along the anterior-posterior axis. The width of the portion of a bone that is affected can often be greater than the width of a tunnel required to gain axis to that portion of the bone.

It is known to provide an instrument for forming a cavity in a bone that is arranged to form a cavity which is larger than the access hole into the bone through which the instrument is inserted. For instance, US-2001/0034526-A1 discloses an expandable reamer for forming a space within a vertebral disc. The expandable reamer includes a pair of opposing blades having an expanded position in which they extend from either side of a shaft assembly and a retracted state in which they are substantially retracted into the shaft assembly. When the blades are in the retracted state the shaft assembly may be inserted into a bore extending into a bone. The blades may then progressively be deployed to the expanded position while the shaft assembly is rotated to form a cavity having a greater maximum diameter than that of the shaft assembly or the bore extending into the bone.

EP-0370204 discloses a drilling device for producing a drilled hole with a recess. A shank is guided within a guide tube into a bore extending into a substrate. The shank further comprises a swing out cutting element which is forced outward from the bore under axial pressure applied to the guide tube in order to form a recess within the bore.

DE-931024 discloses a drilling device for forming a recess in the side wall of a bore extending into a substrate. Axial pressure applied to a guide rod causes a cutting element to extend from the guide rod to form a recess within the side wall of the bore. This document forms the basis for the preamble of claim 1.

FR-2479051 discloses a drilling device for forming a recess in the side wall of a bore extending into a substrate. Twin blades extend from a side wall of the drilling device to form the recess.

EP-0008622 discloses a drilling tool for making undercuts. A drill extends into a bore into masonry. Extendable arms extend from the drill to form secondary drills by means of an axially displaceable sleeve.

US-2005/0240193-A1 discloses a device for creating voids in interior body regions. The device comprises a cutting tip for a tool for creating voids. The cutting tip provides for rotational and translational cutting. An actuator mechanism for deploying the cutting tip converts rotational movement of a wheel into translational movement of a plunger rod.

WO-01/74258-A1 discloses a method and apparatus for forming a cavity in soft tissue or bone. The device comprises a cutting tip member arranged to rotate relative to a guide rod in order to form the cavity.

It is an object of embodiments of the present invention to obviate or mitigate one or more of the problems associated with the prior art, whether identified herein or elsewhere.

The present invention is defined in the appended claims.

According to a first aspect of the present invention there is provided an instrument for forming a cavity within a bone, the instrument comprising: a shaft; a blade having two cutting portions pivotally mounted on the shaft towards a first end of the shaft such that the two cutting portions are arranged on opposite sides of the pivot; a push rod extending within the shaft and coupled to the blade, the push rod being arranged to slide within the shaft to cause the blade to rotate about its pivot; a depth stop coupled to the shaft arranged such that when the shaft is inserted into a bore extending into a bone the depth stop is arranged to engage bone surrounding the bore to limit movement of the shaft into the bore; and a spring coupling the push rod to the shaft; wherein the spring is arranged to resist axial movement of the push rod within the shaft towards the first end of the shaft, the instrument being arranged such that when the depth stop engages bone surrounding a bore, axial force exceeding the spring resistance applied to the push rod causes the push rod to slide within the shaft.

An advantage of the first aspect of the present invention is that the depth stop automatically determines the distance of the cavity from the surface of the bone along the bore. Consequently, a surgeon is able to couple the push rod to a rotary drive to rotate the instrument and apply axial force to the push rod through the rotary drive. The blade remains retracted until the depth stop engages the bone preventing further insertion of the shaft into the bore and the applied axial force exceeds the resistance of the spring. Upon releasing the axial force the spring causes the push rod to retract from the shaft retracting the blade.

The depth stop may comprise a guide sleeve extending around the shaft and coupled to the shaft. The instrument may further comprise a handle disposed about the shaft and arranged to slide over the shaft, the guide sleeve extending over the shaft from the handle such that the shaft can rotate within the handle and the guide sleeve. The guide sleeve may be coupled to the handle such that the length of the cutting guide sleeve extending from the handle is adjustable to adjust the limit of insertion of the shaft into a bore.

The shaft may comprise an end stop positioned towards a second end of the shaft, the end stop being arranged to prevent the guide sleeve from passing over the second end of the shaft. The push rod may extend beyond the end stop and terminates at a coupler arranged to connect the instrument to a rotary drive for rotating the push rod, shaft and blade. The spring may extend between the coupler and the end stop, the spring being arranged to resist an axial force applied to the push rod such that absent of any axial force applied to the push rod the blade is generally aligned with the longitudinal axis of the shaft.

The instrument may further comprise a flange coupled to the portion of the push rod extending from the shaft, the spring being coupled between the flange and the end stop, wherein the spring has a normally biased extended configuration in which the flange is spaced apart from the end stop and a compressed configuration, sliding of the push rod within the shaft being limited by the difference in length of the spring between the extended configuration and the compressed configuration.

The axis of the pivot about which the blade rotates may be perpendicular to the longitudinal axis of the shaft, the blade being arranged to rotate about its pivot on the shaft between a first position in which the cutting portions do not extend transversely beyond the side walls of the shaft and a second position in which the first and second cutting portions extend from the shaft transverse to the longitudinal axis of the shaft.

The cutting edge of the first cutting portion may be rotationally offset about the pivot of the blade on the shaft from a position diametrically opposite the cutting edge of the second cutting portion in the direction of rotation of the blade between the first and the second positions.

An exemplary method of forming a cavity in a bone using an instrument according to an embodiment of the present invention comprises: identifying a required cavity location in the bone; forming a bore in the bone extending to the required cavity location; providing an instrument which comprises a shaft, a blade having two cutting portions pivotally mounted on the shaft towards one end of the shaft such that the two cutting portions are arranged on opposite sides of the pivot, a push rod extending within the shaft and coupled to the blade arranged to slide within the shaft to cause the blade to rotate about its pivot, a spring coupling the push rod to the shaft arranged to resist axial movement of the push rod within the shaft towards the first end of the shaft, and a depth stop coupled to the shaft; inserting the instrument into the bore until the depth stop engages bone surrounding the bore and limits further movement of the shaft into the bore; coupling the push rod to a rotary drive; applying rotary force to the push rod to rotate the shaft, push rod and blade about the longitudinal axis of the shaft; and applying axial force to the push rod exceeding the spring resistance such that the blade is progressively rotated relative to the shaft about its pivot.

Identifying a required cavity location in the bone may comprise locating necrotic bone tissue such that the method is arranged to treat avascular necrosis.

The rate of cutting of the bone is at least in part dependent upon the cutting media (that is, the bone). The rate at which the blade is deployed is determined by the respective axial force applied to the push rod by the rotary drive (that is, the drill) and the hardness of the bone. Consequently, the surgeon is able to obtain a feel for the degree of cutting by judging the axial force required to fully deploy the blade.

The exemplary method may further comprise: providing two or more instruments sharing a common depth stop, the blades of the two or more instruments have a different length measured from the pivot of the blade on the shaft to the end of a first cutting portion; wherein the depth stop comprises a handle arranged to be disposed about the shaft and arranged to slide along the shaft and a cutting guide sleeve arranged to extend over the shaft from the handle, wherein the handle and the cutting guide sleeve are arranged such that the shaft can rotate within the handle and the cutting guide sleeve, and wherein the cutting guide sleeve is coupled to the handle such that the length of the cutting guide sleeve extending from the handle is adjustable; inserting the shaft of the instrument having the smallest blade length through the handle and the guide sleeve; adjusting the length of the cutting guide sleeve extending from the handle such that when the instrument is inserted into the bore the end of the cutting guide sleeve contacts cortical bone surrounding the bore and the blade contacts the end of the bore; forming a cavity in the bone; and replacing the instrument with an instrument having a longer blade length to form a larger cavity in the bone.

Advantageously the cavity may be sequentially enlarged by using instruments with progressively longer blades. However, typically each sequential cut cavity may be tangential to the preceding cavity. That is, after the first spherical cavity is formed, each following larger cavity may be positioned such that its furthest position along the bore is aligned with the side of the preceding cavity. This is achieved by ensuring that for each instrument the length of the shaft plus the fully extended blade is the same, regardless of the length of the blade (that is, length of the shaft from the depth stop to the blade pivot reduces in proportion to the increased length of the blade).

There is also disclosed an instrument for forming a cavity within a bone, the instrument comprising: a shaft; a blade pivotally mounted on the shaft towards a first end of the shaft; a push rod extending within the shaft and coupled to the blade, the push rod being arranged to slide within the shaft to cause the blade to rotate about its pivot; a depth stop coupled to the shaft arranged such that when the shaft is inserted into a bore extending into a bone the depth stop is arranged to engage bone surrounding the bore to limit movement of the shaft into the bore; and a spring coupling the push rod to the shaft; wherein the spring is arranged to resist axial movement of the push rod within the shaft towards the first end of the shaft, the instrument being arranged such that when the depth stop engages bone surrounding a bore, axial force exceeding the spring resistance applied to the push rod causes the push rod to slide within the shaft.

There is disclosed an instrument for forming a cavity within a bone, the instrument comprising: a shaft; a blade having two cutting portions, the blade being pivotally mounted on the shaft towards one end thereof such that the two cutting portions are arranged on opposite sides of the pivot; and a push rod extending within the shaft and coupled to the blade, the push rod being arranged to slide within the shaft to cause the blade to rotate about its pivot.

Advantageously, a blade having two cutting portions can be moved at the end of a shaft between a start position and a finish position. Accordingly, the instrument permits location of the blade through a small diameter tunnel, before being deployed in a desired location to form a cavity in that location. The resulting cavity can be wider than the tunnel through which access is gained to the affected bone tissue.

By deploying a blade having two cutting portions disposed on opposite sides of a pivot point the deployment mechanism may be relatively simple as only a single rotational movement is required, as opposed to certain known instruments in which two blades are independently mounted upon a shaft such that they rotate in opposite directions.

During formation of a cavity in a bone, each cutting portion of the blade separately engages the bone. The action of each cutting portion against the bone results in a force being applied to the instrument along the axis of the shaft which serves to either urge the instrument out of the bore in the bone or to pull the instrument further into the bore. As the cutting portions are arranged on opposite sides of the pivot point, these forces may be balanced in order to provide a resultant neutral axial force, or a predetermined axial force, for instance to keep the instrument positioned firmly against the end of the bore.

Having a single blade with two cutting portions advantageously reduces the diameter of the instrument shaft compared to similar instruments have a single blade with a single cutting portion or two separately deployable blades. This is because the extent to which the blade must rotate in order to form a spherical cavity in the bone is reduced. A spherical cavity is achieved by ensuring that the length of each cutting portion from the blades pivot on the shaft to the tip of the cutting portion is the same.

The instrument can be used to form a cavity in a bone that is rotationally symmetrical about the axis of a tunnel through which the instrument is inserted into the bone. The size of the cavity is dependent on the length of the cutting portions of the blade in the instrument. The shape of the cavity is dictated by the paths that the tips of the cutting portions follow as they rotate about the blade pivot point. A generally round cavity is created by the rotation of the two cutting portions about the blade pivot point and by rotating the shaft about its longitudinal axis concurrently.

During the cutting operation, the instrument is rotated about the axis that is defined by the shaft. During such rotation, the blade can be gradually deployed at a rate that is independent of the speed of rotation of the instrument about the axis of the shaft. Owing to the fact that the blade has two cutting portions, by pivoting the blade through an angle approaching 90°, while holding the shaft against movement relative to the bone, a cavity can be formed in the bone whose shape approaches that of a sphere. By pivoting the blade through a smaller angle, cavities can be formed in the bone whose shape approximates segments of a sphere when the cavity is viewed from one side perpendicular to a plane that contains the axis of the access tunnel.

The blade may be shaped so that it does not protrude unacceptably from either side of the shaft when it is retracted. This can effectively limit the width of the blade. The cutting edges of the blade at the end of each cutting portion should be sufficiently wide that successive sweeps of the blade across the bone tissue when the blade is in use overlap. The leading edge of each cutting portion may also comprise a cutting edge. The end faces, or at least one end face, of the blade can be slightly rounded when the blade is viewed from one side.

The push rod may be pivotally coupled to the blade at a point on the blade which is eccentric of the pivot of the blade on the shaft.

The axis of the pivot about which the blade rotates may be perpendicular to the longitudinal axis of the shaft.

The push rod may be coupled to the blade via a link rod which is pivotally coupled at respective ends to the blade and the push rod.

The blade may be arranged to rotate about its pivot on the shaft between a first position in which a first cutting portion is retracted into a blade slot formed in the end of the shaft and the second cutting portion extends from the end of the shaft and a second position in which the first and second cutting portions extend from the shaft transverse to the longitudinal axis of the shaft. The blade may rotate through no more than 90° between the first position and the second position.

The leading edges of the first and second cutting portions as the blade rotates from the first position to the second position may comprise cutting edges. The cutting edge of the first cutting portion may be rotationally offset about the pivot of the blade on the shaft from a position diametrically opposite the cutting edge of the second cutting portion in the direction of rotation of the blade between the first and the second positions.

The blade may further comprise a shoulder portion arranged to contact the push rod or the link rod as the blade rotates towards the second position to prevent further rotation beyond the second position of the blade.

The end of the second cutting portion remote from the pivot of the blade on the shaft may be curved.

The length of each cutting portion from the pivot of the blade on the shaft to the tip of the cutting portion may be the same.

There is also disclosed an instrument set comprising: two or more instruments as described above; a handle arranged to be disposed about the shaft of one of the instruments and arranged to slide along the shaft; and a cutting guide sleeve arranged to extend over the shaft from the handle; wherein the shaft can rotate within the handle and the cutting guide sleeve, and wherein the cutting guide sleeve is coupled to the handle such that the length of the cutting guide sleeve extending from the handle is adjustable.

The blades of the two or more instruments may have a different length measured from the pivot of the blade on the shaft to the end of the first or the second cutting portion.

For each instrument the sum of the length of shaft measured between the pivot of the blade on the shaft and the end of the shaft remote from the blade and the length of either the first or the second cutting portion may be the same.

Instruments in accordance with embodiments of the present invention can be made from materials that are commonly used in the manufacture of surgical instruments. Examples of such materials include metals such as certain stainless steels, and polymers such as polyolefins (especially polyethylenes and polypropylenes), polyamides, polyesters and polycarbonates. It will generally be preferred for the shaft and the rod and the blade, at least, to be formed from metallic materials.

Embodiments of the invention will now be described by way of example with reference to the accompanying drawings, in which:
Figure 1 is an isometric view of a manually powered instrument for forming a cavity within a bone in which deployment of a cutting blade is related to the rate of rotation of the instrument;
Figure 2 schematically illustrates the deployment of a blade forming part of the instrument of figure 1;
Figure 3 schematically illustrates the deployment of a blade forming part of an instrument for forming a cavity within a bone in accordance with an embodiment of the present invention;
Figure 4 is an isometric view of an instrument for forming a cavity within a bone in accordance with an embodiment of the present invention;
Figure 5 is a side view of a shaft and a rod extending through the shaft, which form part of the instrument of figure 4;
Figure 6 is a side view of the shaft and rod of figure 5 rotated through 90°;
Figure 7 is an isometric view of a first end of the shaft and rod of figure 5;
Figure 8 is a cross sectional view of part of the shaft and rod of figure 5 along the line 8:8 in the direction of the arrows;
Figure 9 is an isometric view of a second end of the shaft and rod of figure 5;
Figure 10 is a partial cross section view of the second end of the shaft and rod illustrating a blade deployment mechanism in the orientation illustrated in figure 6;
Figure 11 is an isometric view of the second end of the shaft and rod of figure 5 generally in the orientation of figure 10 in which a blade has been deployed;
Figure 12 is a cross section view of a handle forming part of the instrument of figure 4; and
Figure 13 is an isometric exploded view of the handle of figure 12.

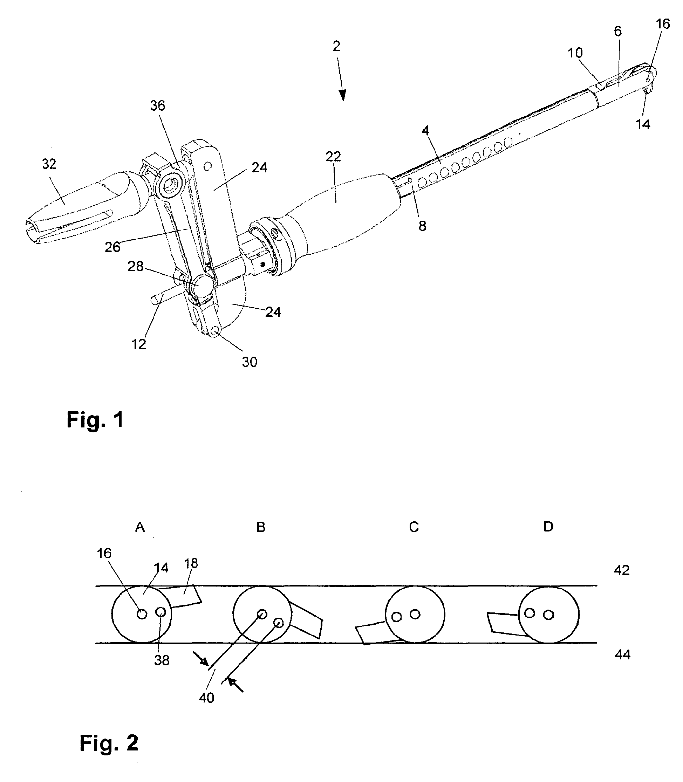

Referring to the drawings, figure 1 shows a cutting instrument 2 adapted to be inserted into a tunnel, which extends into a bone. For instance, the tunnel may extend medially through the neck of a femur, from the lateral cortex into the head of the femur. The tunnel is formed using a rotating cutting instrument such as a drill or a reamer or using a combination of cutting instruments. The tunnel may extend into the head sufficiently so that the end of the instrument is located in a region of the head in which the bone tissue is defective, for example as a result of loss of blood circulation to the bone tissue, such as when the patient is suffering from avascular necrosis.

The instrument comprises a hollow shaft 4 having a blade end 6 and a handle end 8. The shaft has a blade slot 10 formed in its sidewall at the blade end 6. A rod 12 extends through the shaft 4. A blade 14 is mounted to pivot within the blade slot 10 on a central pivot pin 16. The blade has a cutting portion 18 and a body portion 20. The central pin 16 passes through a hole in the body portion 20. The length of the blade slot 10 in the shaft 4 is slightly greater than the length of the blade 14 measured from the central pin 16 to the end of the blade 14, and the central pin 16 is located in the slot 10 at a point which is proximal the blade end 6 of shaft 4.

The rod 12 is connected at one end to the blade 14 by means of a pin (not visible in figure 1, but described in greater detail in connection with figure 2), at a point on the blade body portion 20 which is on the same side of the pivot pin 16 as the cutting portion 18. The pin by which the rod 12 is connected to the blade 14 allows the blade 14 to rotate about central pin 16 relative to the rod 12 (and the shaft 4). The blade 14 can be made to pivot about the central pin 16 by moving the rod 12 axially within the shaft 4. Blade 14 may be rotated from a start position in which it extends distally from the blade end 6 of the shaft 4, through an arc of approximately 160° until it is retracted into the blade slot 10 in a finish position. In use for forming a cavity in a bone, the instrument 2 is inserted into a bore extending into the bone with the blade 14 in the start position. The instrument is then rotated about the axis of shaft 4 while progressively rotating the blade 14 until the blade 14 is in the finish position at which point the instrument 2 may be retracted from the bore.

The shaft 4 has a handle 22 formed on it at the handle end 8. The handle 22 is wider than the shaft 4. Handle 22 is allowed to rotate about shaft 4 so that a user can grip it comfortably in any position. The rod 12 extends beyond the end of the handle 22. A lever support arm 24 extends from the handle 22. The lever support arm 24 is formed integrally with the handle 22.

The instrument includes a control arm 26. The control arm 26 has an opening 28 extending through it in which the rod 12 is received, and held by means of a fastener. The control arm 26 is pivotally connected at one end to the lever support arm 24 by pivot pin 30. Pivotal movement of the control arm 26 relative to the lever support arm 24 causes the rod 12 to move axially relative to the handle 22 and the shaft 4.

The control arm 26 has a knob 32 at its free end, which is free to rotate relative to the control arm 26. The knob 32 is mounted on the control arm 26 on a fastener that extends through an aperture in the control arm 26.

Operation of the instrument 2 involves rotation of the instrument 2 about the axis of the shaft 4. This rotational movement is achieved by gripping the instrument 2 using the handle 22 which is allowed to rotate about shaft 4. Rotational movement of the instrument about the axis of the shaft 4 is then imparted using the knob 32, which is spaced from the axis of the shaft 4, by applying a torsional force to the knob 32 relative to the handle 22. Both handle 22 and knob 32 remain stationary in the operator's hands, with the knob 32 being rotated in a circle about the shaft 4. The cutting edge 34 on the blade 14 shaves bone tissue contacted by the blade 14 when the instrument 2 is rotated.

The blade 14 can be moved pivotally relative to the shaft 4 between the start and finish positions by moving the rod 12 within the shaft 4. This can be achieved by moving the control arm 26 about the pivot 30 where it is connected to the lever support arm 24.

An incremental drive causes the rod 12 to translate along the shaft 4 when the shaft 4 and rod 12 are rotated. The incremental drive is provided by the lever support arm 24, which also extends from the shaft 4, in a direction that is opposite to the portion of the lever support arm 24, which is pivotally connected to the control arm 26. A drive rod 36 is pivotally connected to the lever support arm 24. The drive rod 36 has a thread formed on its outer surface. Knob 32 has a threaded bore formed in it, so that it can receive the end of the threaded drive rod 36. Accordingly, as the knob 32 is rotated relative to the drive rod 36 and to the control arm 26, the knob advances along the drive rod 36. Advancement of the knob 32 along the drive rod 36 causes the control arm 26 to rotate about its pivot 30. As discussed above, rod 12 is coupled to the control arm 26 by fastener 28. Therefore pivoting of the control arm 26 causes the rod 12 to slide within shaft 4.

Figure 1 shows the instrument 2 when the blade 14 is part way between the start and finish positions. Rotation of the blade 14 from the start to the finish position is achieved by rod 12 sliding towards the handle end 8 of shaft 4. The knob 32 has been rotated so that it has advanced part way along the drive rod 36. Consequently, the rod 12 has been retracted through the shaft 4 towards the handle end 8 of shaft 4, to cause the blade 14 to be deployed.

The instrument 2 illustrated in figure 1 is intended to create a spherical cavity within a bone, and is particularly suited for forming a spherical cavity within the head of a femur accessed through a medial bore. Deployment of the blade 14 that cuts into the bone to form the cavity is linked directly to the rotation of the crank handle (formed from knob 32, control arm 26 and lever support arm 24 extending from the handle 22 transverse to the longitudinal axis of shaft 4). The blade 14 has a single cutting portion 18 that rotates about central pin 16 from a start position, which extends from the blade end 8 of shaft 4 along the longitudinal axis of the shaft 4, through the deployed position illustrated in figure 1 to a finish position contained within blade slot 10. Alternatively, during a cutting operation the blade may rotate about central pin 16 in the opposite direction.

However, the instrument 2 illustrated in figure 1 suffers from a number of limitations, which are inherent in its design. Firstly, in order to generate sufficient torque for the cutting portion 18 to cut bone, the crank handle must have a significant radial sweep about shaft 4. In practice in order to form a cavity in the head of a femur in which the access tunnel extends medially through the femoral neck, the instrument must enter the femur at an angle of approximately 45° to the long axis of the femur. Therefore, in order to prevent the crank handle impinging on soft tissue of the patient's leg during rotation of the crank handle, shaft 4 must extend a considerable distance from the patient before being coupled to the handle 22.

A second limitation is that in order to cut bone the surgeon must apply a large tangential force to the crank handle. This can cause the shaft of the instrument to move in a conical fashion. Conical movement can cause the patient's leg to move and increases the potential for misalignment or damage to the bone. The large radial sweep of the crank handle and the consequent length of the shaft extending from the patient's leg exacerbate the potential for conical movement.

A further limitation is that as the rotation of the instrument and the deployment of the blade are linked, the surgeon cannot easily regulate the progressive deployment of the blade. While it is possible to ensure that the progression of the blade about its pivot point is independent of the rotation of the instrument within the bore, in order to achieve this, the rate of rotation of knob 32 relative to the control arm 26 as the control arm 26 is rotated about the shaft 4 must be varied. This can be difficult to achieve in practice. It can be desirable for the rate of deployment of the blade to be varied to take account of changes in bone density for instance due to sclerotic bone. It would be desirable for the surgeon to be able to reduce the rate of deployment of the blade when dense bone mass is encountered, that is at times at which a high torque must be applied to the shaft.

A further limitation is that as one cutting edge engages the bone, an unbalanced axial load is created that tends to displace the instrument during cutting of a cavity. The axial load may serve to force the instrument from the bone. Consequently, the surgeon must counter this axial load while simultaneously turning the crank handle, again increasing the potential for misalignment and damage to the bone. It can be difficult to accurately control the depth of the instrument within the bore, resulting in difficulty in forming a cavity at the appropriate position within the bone.

The blade 14 rotates about central pivot pin 16 in a plane that includes the longitudinal axis of the shaft 14. Referring now to figure 2, this schematically illustrates the change in the rotational position of the blade 14 about central pin 16 as the cutting portion 18 of the blade is deployed. Each separate view (A-D) of the blade 14 is viewed along the axis of the pivot pin 16, (perpendicular to the plane of rotation of the blade 14). For each view of the blade 14, the blade end 6 of the shaft 4 is to the right. The horizontal lines indicate the outer diameter of the shaft 4. That is, when the cutting portion 18 is entirely contained between the horizontal lines, the blade 14 is either retracted in the blade slot 10 (views C and D) or extends beyond the blade end 6 of the shaft 4 (views A and B).

As discussed above, blade 14 is coupled to the push rod 12 by a second driving pivot pin, illustrated in figure 2 as 38 (rod 12 is not illustrate in figure 2, but it will be appreciated that it extends from driving pin 38 towards the left of the figure). There may be a link rod between the driving pin 38 and the rod 12 in order to prevent movement of the blade 14 shifting the axial alignment of the rod 12 within the shaft 4.

View B illustrates the blade 14 in its starting position, within the cutting portion 18 extending distally of the blade end 6 of the shaft 4. View C illustrates the blade 14 in its final position contained within the blade slot 10. As only a single cutting portion of the blade is provided, it will be appreciated that the blade must rotate through approximately 160° in order to cut a full spherical cavity in the bone surround the blade end of the shaft. The required rotation is less than 180° because the blade 14 has a width in the cutting portion 18 between the leading cutting edge 34 and the trailing edge. The end of the blade 14 in the cutting portion 18 contacts the end of the bore in the start position, which means that the cutting edge 34 does not need to be rotated through this portion of the arc to cut a full sphere. Cutting is achieved when the blade is partially or fully deployed from the start position by the blade being rotated around the longitudinal axis of the shaft (that is, the plane of cutting rotation of the blade 14 is perpendicular to the plane illustrated in figure 2). The extent of rotation of the blade about central pin 16 to cut a spherical cavity can be seen in figure 2 by the relative movement of the driving pin 38 between views B and C.

Because of the crank drive of shaft 4, the radial offset 40 between the central pin 16 and the driving pin 38 must be relatively large to ensure that the driving pin does not pass over centre (that is, as illustrated in figure 2, the driving pin 38 does not rise above the central pin 16 at the limits of its travel). The driving pin 38 passing over centre is illustrated in views A and D at the start and finish of the rotation of the blade 14 respectively. This is because rotation of the crank handle at a constant rate causes the rod 12 to progress through the shaft 4 at a constant rate. However, when the driving pin 38 is approaching dead centre, rod 12 extending along the shaft 4 at a constant rate causes the blade 14 to rotate more quickly increasing the risk of passing over centre. If the radial offset 40 is increased the then the rate of rotation of the blade 14 is reduced given the same rate of axial movement of rod 12. If the driving pin 38 passes over centre, the instrument can be locked in its open or closed position. As the driving pin approaches dead centre, the axial movement of the push rod tends to zero (due to the limit of travel of the drive rod 36 being reached) and thus no force is applied to the driving pin 38 to prevent the pin from passing over dead centre, for instance if it is perturbed by the conical motion of the instrument.

Central pin 16 extends through the blade 14 and into the shaft 4 on either side of the blade. The large extent of rotation of the driving pin 38 about the central pin 16 provides another reason why the radial offset 40 must be large, in order to provide sufficient clearance between the push rod 12 and the central pin 16 where the push rod 12 is coupled to the driving pin 38 (or sufficient clearance between the central pin 16 and a link rod coupling the driving pin 38 to the push rod 12).

At the start and finish rotational positions of blade 14 illustrated in views B and C, the angle subtended between a line extending between the central pin 16 and the driving pin 38 and the longitudinal axis of the rod 12 is small. This is due to the requirement for the blade to rotate through approximately 160° without the driving pin passing over centre. Therefore, the component of the axial force applied to the driving pin by the push rod that serves to rotate the blade (the component of the axial force acting through the driving pin perpendicular to the line connecting the two pins) when the blade is close to its start and finish positions is small. In order to ensure that sufficient torque is applied to the blade to rotate the blade about the central pin such that bone resistance is overcome, without requiring the surgeon to apply an excessive force to the crank handle, again the radial offset must be relatively large.

The large required radial offset 40 means that the diameter of shaft 4 must similarly be large to accommodate the sweep of the driving pin 38 about the central pin 16 within the diameter of the shaft 4 indicated by the horizontal lines 42, 44. This consequently requires a large access tunnel extending into the bone in order to receive the instrument before a spherical cavity can be bored. It is known that larger access tunnels into a bone increase the risk of damage to the bone. In the case of an access tunnel extending into the head of a femur, this increases the risk of damage to the femoral neck or subtrochanteric fracture.

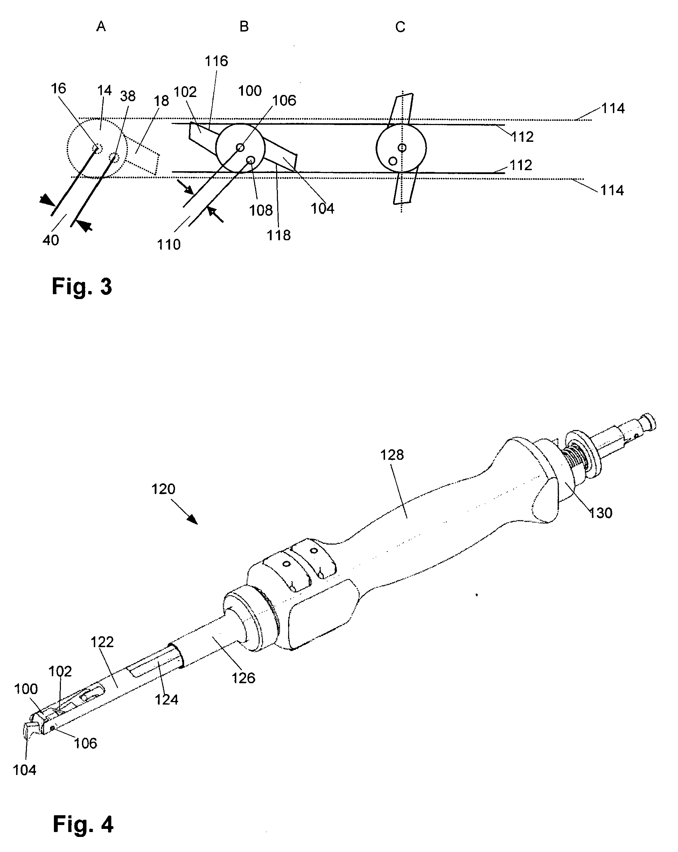

In accordance with embodiments of the present invention, an instrument for forming a cavity in a bone is provided having a blade with two cutting portions, which overcomes certain of the limitations of the instrument described above in connection with figures 1 and 2. Referring to figure 3, this schematically illustrates in views B and C a blade 100 having first and second cutting portions 102, 104 in accordance with an embodiment of the present invention. For comparison, the blade 14 of figure 2 in the start position is again illustrated in view A. The orientation of blades 14 and 100 in figure 3 with respect to the remainder of an instrument for forming a cavity in a bone is the same as for figure 2. In particular, blades 14, 100 are arranged to rotate about central pins 16, 106 respectively. The rotation of blades 14, 100 is driven by an axial force provided by a push rod (not illustrated) coupled to driving pins 38, 108.

In order to form the same spherical cavity, blade 14 must rotate through approximately 160° as described above in connection with figure 2. However, blade 100 need only rotate through less than 90°, for instance approximately 80°. View B illustrates blade 100 in its start position and view C illustrates blade 100 in its finish position. Due to the reduction in the angle through which blade 100 must rotate, the position of the driving pin 108 in its start and finish position can be moved. By comparison of views A and B (which illustrate blades 14 and 100 respectively in their start positions) it can be seen that driving pin 108 is positioned such that the angle subtended between a line extending between the central pin 106 and the driving pin 108 and the longitudinal axis of the push rod (extending horizontally to the left of figure 3) is large compared to the equivalent angle for blade 14 in view A. Consequently, in order to provide the same torque to the blade 100 such that the blade can overcome the same bone resistance as for blade 14, the radial offset 110 between pins 106 and 108 can be reduced relative to radial offset 40 for blade 14.

As the radial offset 110 is smaller, the diameter of the shaft through which the push rod passes may also be smaller as indicated by the gap between solid lines 112 relative to the corresponding shaft diameter indicated by dashed lines 114 for blade 14. This consequently requires a smaller access tunnel extending into the bone in order to receive the instrument before a spherical cavity can be bored, which reduces the risk of damage to the bone.

Furthermore, as the angle subtended between a line extending between the central pin 106 and the driving pin 108 and the longitudinal axis of the push rod is relatively large for blade 100 in both the start and finish positions (views B and C) the possibility of the driving pin 108 passing over centre is eliminated.

The tendency for the instrument to drift during cutting of a cavity in a bone is reduced for an instrument including a blade 100 having two cutting portions. This is because the forces acting along the longitudinal axis of a push rod coupled to the blade, generated by the blade reacting on the bone can be made to be symmetrical. Blade 100 comprises first and second cutting portions 102, 104. As the blade 100 rotates in a clockwise direction from its start position (in the view of figure 3) cutting edges 116 and 118 respectively engage the bone. Cutting edges 116 and 118 are arranged such that they extend from the central pin 106 in opposite directions as illustrated in figure 3. Cutting edge 116 will generate a force component aligned with the cutting rod longitudinal axis that serves to force the instrument out of the cavity. Cutting edge 118 will generate a force component aligned with the cutting rod longitudinal axis that serves to draw the instrument into the cavity. These force components will cancel one another out. Note that it is not necessary that each cutting edge 116, 118 is exactly aligned with a radial line extending from central pin 106, so long as each cutting blade is arranged relative to the radial line in the same way.

Alternatively, it can be desirable to rotationally offset one of the cutting edges 116, 118 in order to generate a small axial force, for instance a small axial force which serves to pull the instrument into the cavity, the pulling force being resisted by a portion of the instrument bearing against cortical bone surrounding the bore, as will be described below in connection with figure 4. This pulling force further reduces the tendency of the instrument to drift during cavity cutting. The portion of the instrument bearing against the cortical bone ensures that the shaft remains in the same axial position during cutting. The net force acting on the instrument along the axis of the shaft is reduced to a minimum. The pulling force can be achieved by shifting the radial position of the first cutting edge 116 relative to the second cutting edge 118 by a small angular amount in an anticlockwise direction (in the plane illustrated in figure 3).

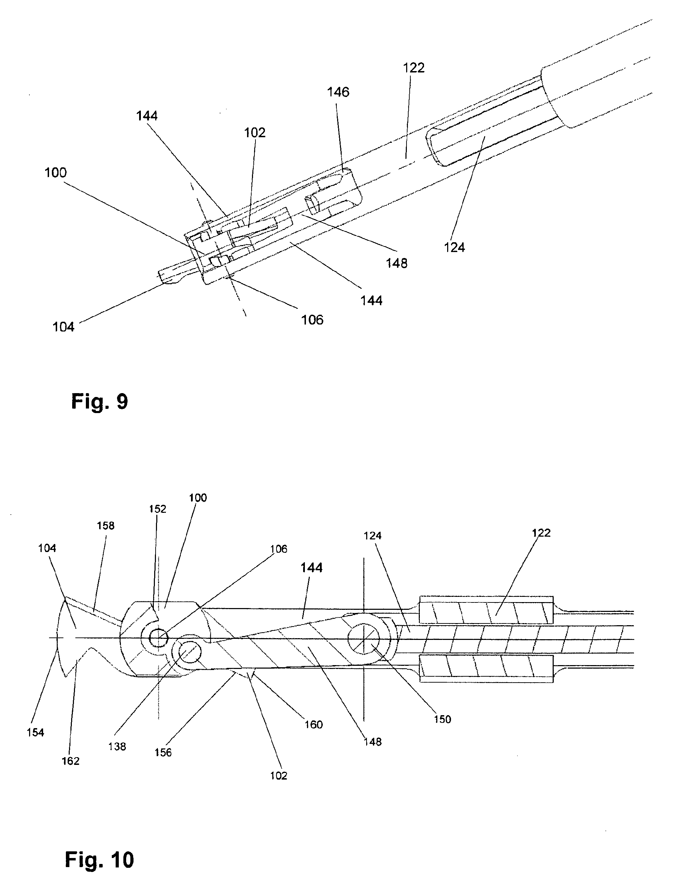

Referring now to figure 4, this illustrates an instrument 120 for forming a cavity within a bone in accordance with an embodiment of the present invention. The instrument 120 incorporates a blade 100 having two cutting portions 102, 104 of the form schematically illustrated in figure 3. It will be appreciated that the shape of the blade 100 for a practical embodiment of the present invention may differ from that illustrated in figure 3. In particular, one embodiment of the blade is illustrated in greater detail in figure 10, and is described below. The instrument illustrated in figure 4 is suitable for forming a similar spherical cavity to that formed by the instrument of figure 1, however the cavity may be formed within a portion of a bone access through a smaller bore due to having a blade generally in the form of that illustrated in figure 3.

The instrument 120 further comprises a tubular shaft 122 and a push rod 124, which is mounted within the shaft 122 such that it can slide parallel to the longitudinal axis of shaft 122. Rod 124 is coupled to blade 100 such that axial movement of the rod 124 causes blade 100 is rotate (as will be described in greater detail below in connection with figure 10).

Instrument 120 further comprises a cutting guide sleeve 126 and a handle 128 coupled to the cutting guide sleeve 126. The cutting guide sleeve 126 and handle 128 are arranged to slide over shaft 122 by passing over the shaft 122 from the blade end. Handle 128 is prevented from sliding over the end of shaft 122 remote from blade 100 by an end stop130. The cutting guide sleeve 126 is coupled to the handle 128 such that the length of the cutting guide sleeve 126 passing over the shaft 122 is adjustable. This allows the end of the cutting guide sleeve 126 remote from the handle 128 to limit the insertion of the instrument 120 into a bore extending into a bone by contacting cortical bone surrounding the bore (the bore being sized to only admit the shaft 122 and not the cutting guide sleeve 126). Alternatively, part of the guide sleeve may be inserted into the bore, depending upon the relative sizes of the bore, the instrument shaft and the guide sleeve. For instance, the guide sleeve 126 may have a reduced thickness portion ending at shoulder portion or flange such that the reduced thickness portion may be inserted into the bone until the shoulder or flange rests against cortical bone. The cutting guide sleeve 126 in combination with the handle 128 serve as a depth stop such that as the instrument is inserted into a bore extending into a bone the position of the cavity to be bored is accurately determined according the position of the sleeve 126 relative to the shaft 122. The depth stop is length adjustable by adjusting the length of the sleeve 126 extending from the handle 128. The end stop 130 is coupled to the shaft 122 and prevents axial pressure applied to the instrument from sliding the shaft 122 further through the handle 128 when the depth stop contacts the edge of the bore or the surrounding bone.

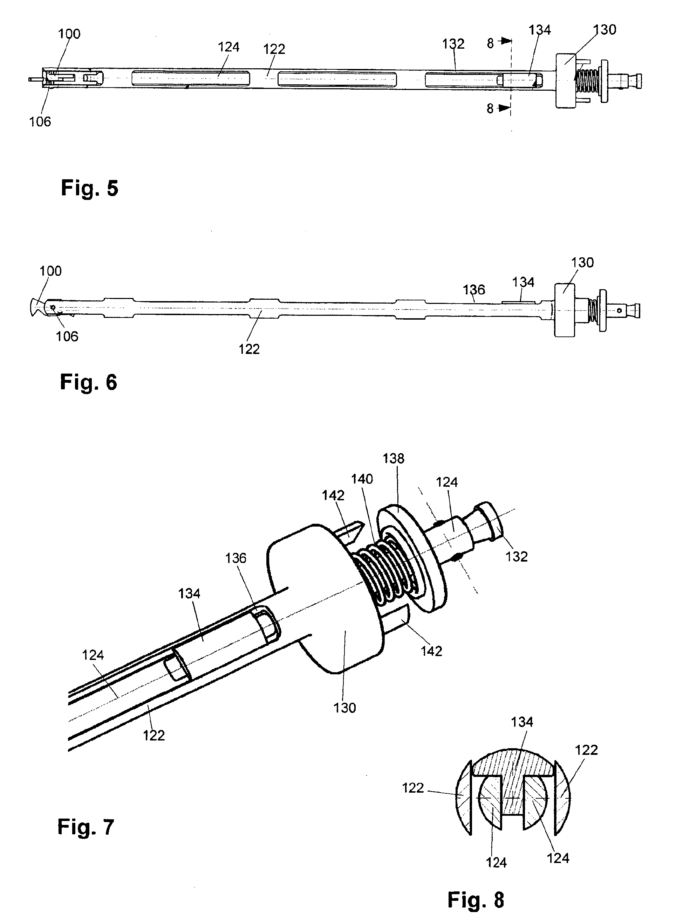

Figures 5 and 6 illustrate in two planes the cavity cutter part of the instrument 120 (that is, instrument 120 with the cutting guide sleeve 126 and the handle 128 removed) in order to illustrate in greater detail the arrangement of the shaft 122 and rod 124. Figure 6 illustrates the cavity cutter portion of the instrument 120 after it has been rotated by 90° around the longitudinal axis of the shaft 122. Shaft 122 is has a tubular section and has openings cut into it through which the rod 124 can be seen in figure 5.

Figure 7 illustrates in an isometric view a first end of the cavity cutter portion of the instrument 120 illustrated in figures 5 and 6. The end of the instrument illustrated in figure 7 is the end remote from the blade 100. Rod 124 protrudes from the end of shaft 122 through the end stop 130 and terminates in a standard Hudson coupler 132. It will be appreciated that any alternative known form of coupling may be used. The Hudson coupler 132 allows the instrument 120 to be coupled to a power tool for rotating the rod 124 about its longitudinal axis. The coupler 132 is generally wedge shaped to provide a positive engagement with the power tool. However, in alternative embodiments the coupler 132 may be removed to allow the push rod to be detached from the power tool to prevent the shaft from being retracted from the bore by pulling on the power tool. This prevents an oval shaped cavity from being formed by retracting the instrument while continuing to rotate the shaft.

As noted above, rod 124 can slide within the shaft 122. Rod 124 is coupled to the blade 100 (as will be described in greater detail below) such that axial movement of the rod 124 causes the blade 100 to rotate around central pivot 106. Blade 100 is in turn coupled to the shaft 122 by central pivot pin 106 passing through the blade 100 and opposing sides of the shaft 122. The sides of rod 124 include flattened portions arranged to engage corresponding flattened portions within the internal side walls of shaft 122. Consequently, rotation of the rod 124 driven by the power tool via Hudson coupler 132 causes the shaft 122 to also rotate. The cutting guide sleeve 126 and the handle 128 are arranged to be mounted on the shaft 122 so that the handle may be held firm by a surgeon while the shaft 122 rotates. Rod 124 is also coupled to shaft 122 via a torque key 134 illustrated in figures 5, 6 and 7 and illustrated in cross section in figure 8. In cross section, torque key 134 comprises a T-shaped wedge which is inserted into a slot in rod 124. The cross piece of the wedge fits into a window 136 cut into shaft 122. The arrangement is such that torque key 134 can slide within window 136 parallel to the longitudinal axis of shaft 122 as the rod 124 slides axially within shaft 122 while still transferring torque to the shaft 122 from the power tool. Thus, the rod 124 is coupled to the shaft at positions proximal to both ends of the shaft 122.

A flange 128 is coupled to the rod 124 proximal to the Hudson coupler 132. A spring 140 extends between the flange 138 and the end stop 130 of the shaft 122. Spring 140 is normally biased to space apart the end stop 130 and the flange 138. Arms 142 extend from the end stop 130 part way towards the flange 138. By applying an axial force to the Hudson coupler 132 while holding the shaft 122 in position (in particular, by holding handle 128 firm, but also by the end of the cutting guide sleeve 126 contacting cortical bone around a bore into the bone), the spring 140 can be compressed. This allows rod 124 to slide within the shaft 122 to deploy blade 100, as will be described in greater detail below in connection with figure 10. In particular, an axial force applied to coupler 132 causes the instrument to be inserted into the bore extending into the bone. The spring 140 prevents the blade 100 from being deployed until the depth stop (that is, the end of the cutting guide sleeve 126 or an enlarged portion or flange extending from the sleeve) impinges upon the bone. End stop 130 bears against the end of handle 128, which in turn transfers axial force to the guide sleeve 126, which in turn impinges on the bone surrounding the bore. Further axial force overcomes the resistance of the spring 140 causing the push rod 124 to slide axially within the shaft 122 to deploy the blade 100.

The arrangement of spring 140 and legs 142 is such that the axial movement of the rod 124 within the shaft 122 is limited. When no axial force is applied to overcome the resistance of the spring 140, as illustrated in figure 7, the blade 100 is in the start position schematically illustrated in view B of figure 3. When the spring 140 is compressed such that rod 124 slides within shaft 122, the blade moves to its finished position schematically illustrated in view C of figure 3. In order to allow the instrument 120 to be retracted from a bore extending into a bone, once the axial force is removed from the Hudson coupler 132, spring 138 returns to its normally biased extended position illustrated in figure 7 causing blade 100 to return to its starting position in which the cutting portions 102, 104 extend from the central pivot pin 106 substantially parallel to the longitudinal axis of shaft 122.

The cutting guide sleeve 126 is arranged such that when the instrument 120 is fully inserted into the bore into a bone and cutting of a cavity is ready to begin the end of the cutting guide sleeve 126 remote from handle 128 rests against cortical bone around the bore. Consequently, when an axial force is applied to the Hudson coupler 132, the cutting guide sleeve 126 resists the axial force and prevents the shaft 122 from moving in the direction of the axial force. This ensures firstly that the instrument 120 is not forced further into the bore, and secondly that the axial force compresses the spring 140 causing the rod 124 to slide within shaft 122 causing the blade 100 to deploy.

Unlike the instrument 2 illustrated in figure 1, given that rotation of the rod 124 is driven by a power tool and not a crank handle, no tangential force is applied to the instrument 120. The only forces applied to the instrument 120 are an axial force and torque about the longitudinal axis of the shaft 122. Consequently, this lack of tangential force reduces the tendency towards conical movement exhibited by instrument 2. Furthermore, as there is no crank handle, the overall extent of the instrument 120 extending from the patient's body can be made shorter (by reducing the length of shaft 122 and rod 124) as there is no risk of impinging on soft tissue of the patient's body. Because instrument 120 is shorter than instrument 2, instrument 120 can be more controllable.

Referring now to figure 9, this is an isometric view of part of instrument 120 showing the blade end of shaft 122. Blade 100 is shown pivotally mounted on central pivot pin 106 which extends between arms 144 which comprises side extensions of shaft 122. Arms 144 define the sides of blade slot 146. Rod 124 is shown mounted within shaft 122 and able to slide within shaft 122. Rod 124 is coupled to blade 100 via link rod 148. Link rod 148 is pivotally coupled at each end to the blade 100 and rod 124 respectively (the pivotal coupling of link rod 148 is not visible in figure 9). Figure 9 illustrates blade 100 in its start position with the second cutting portion 104 extending from the shaft 122 generally along the longitudinal axis of the shaft 122 and first cutting portion 106 being contained within the blade slot 146. Blade 100 can be deployed such that it rotates to the finish position by rod 124 sliding towards the blade end of shaft 122. Sliding movement of rod 124 is translated to rotational movement of blade 100 by link rod 146.

Referring now to figure 10, this illustrates in a partial cross sectional side view the blade end of instrument 120. The closest arm 144 has been removed in order to illustrate the deployment mechanism for blade 100. Blade 100 is illustrated in its start position. Link rod 148 is coupled to blade 100 and rod 124 by driving pin 138 and pivot pin 150 respectively. It can be seen that as rod 124 slides towards the blade end of instrument 120 (to the left in figure 10) due to the axial force applied to the Hudson coupler 132, link rod 148 will also move towards the blade end. Central pivot pin 106 is in line with the longitudinal axis of the shaft 122 and the longitudinal axis of push rod 124, whereas driving pin 138 is offset from the longitudinal axis (towards the bottom in figure 10). Therefore, movement of link rod 148 towards the blade end causes blade 100 to rotate clockwise in the view illustrated in figure 10.

As discussed above, movement of rod 124 is limited by spring 140, flange 138 and arms 142. In figure 10 the rod 124 is at the limit of its travel away from the blade end (under the action of spring 140 in its normally biased extended configuration in the absence of any external axial load applied to rod 124). Further rotation of blade 100 in an anticlockwise direction is prevented by the extend of travel of rod 124 away from the blade end of the instrument. Rotation of blade 100 in a clockwise direction is limited by the extent to which rod 124 can move towards the blade end, which is in turn limited by flange 138 contacting arms 144.

Blade 100 further comprises shoulder portion 152. As shown in the isometric view of figure 9, blade 100 comprises a central thicker portion through which the central pin 106 passes, and thinner cutting portions 102, 104. Shoulder portion 152 comprises the transition between the central portion of blade 100 and the first cutting portion 102. The reduced thickness of the blade 100 in the first cutting portion 102 up to the edge of shoulder 152 ensures that the blade does not impinge on the motion of the link rod as the blade rotates. Further clockwise rotation of the blade 100 is limited by the flange 138 contacting arms 142, as described above in connection with figure 7.

In order to create a cavity in a bone, instrument 120 is inserted into a bore, which has been drilled into the bone and passes through the area where the cavity is required. Second cutting portion 104 has a curved end 154, which is arranged to contact the end of the bore limiting the insertion of the instrument into the bore. The radius of curvature of the curved end of the second cutting portion may be substantially the same as the distance from the pivot of the blade on the shaft to the end of the second cutting portion. Typically this will be the case for the smallest cavity cutter. However, for enlarging the cavity with a larger cavity cutter, the radius of curvature of the curved end of the second cutting portion will be less than the distance from the pivot of the blade on the shaft to the end of the second cutting portion. This reduction in curvature is arranged to ensure that the blade butts up against the end of the bore, rather that cutting into the end of the bore.

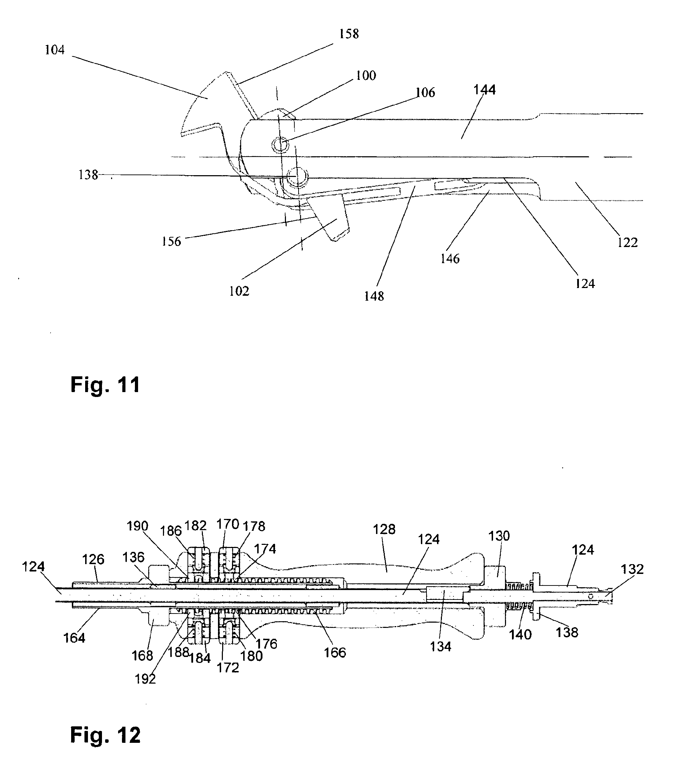

The length of the cutting guide sleeve 126 extending along shaft 122 from handle 128 may then be adjusted such that it contacts the cortical bone around the bore. This provides a reference so that applying an axial force to Hudson coupler 132 does not force the instrument 120 further into the bore. The adjustment of the cutting guide sleeve 126 is described in greater detail below in connection with figure 12.

Applying a torque to Hudson coupler 132 causes the rod 124, shaft 122, link rod 148 and blade 100 to rotate about the longitudinal axis of the shaft 122, while the handle 128 (and therefore also the cutting guide sleeve 126) is held firm by the surgeon. Rotation of blade 100 around the longitudinal axis of shaft 122 causes curved end 154 of the second cutting portion to rotate while in contact with the end of the bore, which serves to form a smooth curve in the end of the bore, if any irregularities are present in the end of the bore. Additionally, curved end 154 allows the surgeon to locate the end of instrument 120 at the end of the bore without damaging the subchondral bone. When the cavity cutter is inserted into the bone, curved end 154 provides a non-cutting datum for determining that the shaft 122 has been fully inserted. Once at the correct depth, the length of the cutting guide sleeve 126 is adjusted until it rests against cortical bone and end stop 130 rests against the handle 128. Consequently, further pushing along the axis of the instrument will not force tip 154 further into the bone.

While driving the rotation of the rod 124 with the power tool, the surgeon may apply an axial force to the power tool which in turn transfers the axial force to rod 124 causing it to move towards the blade end of the instrument. As discussed above, this axial movement of rod 124 causes blade 100 to rotate in a clockwise direction in the view illustrated in figure 10 around central pin 106. Consequently, cutting edges 156 and 158 on the first and second cutting portions 102, 104 respectively are brought into contact with the bone forming the sides of the bore. This causes bone to begin to be cut away to form the cavity. As blade 100 continues to rotate, the end 160 of the first cutting portion 102 also comes into contact with the sides of the cut cavity. Second cutting portion 104 has a hook shaped trailing edge 162 which assists in removing abraded bone due to the volume of metal in the cavity being reduced. Clearance is provided behind the blade for the cut bone fragments to collect in.

The rate of rotation of blade 100 about central pin 106 is independent of the rate of rotation of blade 100 about the longitudinal axis of the shaft 122 and is controlled by the degree of axial load applied to the rod 124 using the power tool. This allows the surgeon to control the cutting rate according to the density of the bone in the same way that the cutting rate of a conventional drill may be controlled by adjusting the axial force applied to the drill while the drill rotates at a constant rate. Additionally, the surgeon is able to back off the blade (moving it in an anticlockwise direction in the view of figure 10) without adjusting the speed or direction of rotation of the shaft 122, should this be required.

In accordance with certain embodiments of the present invention, there may be several cavity cutters provided with a single handle 128 and cutting guide sleeve 126. The blade 100 of each cavity cutter used to create a cavity may be progressively larger allowing a spherical cavity to be cut in incremental steps starting with the smallest cutter and continuing until the required size of cavity is achieved. The length of the cutting guide sleeve only needs to be adjusted once when coupled to the smallest cavity cutter, with the same length of the cutting guide being used with each following cavity cutter to ensure that the cavity is formed at the correct location. Each subsequent cavity cutter has a similar curved end 154 at the end of the second cutting portion 106 of blade 100 to avoid damaging the bone when the new cavity cutter is inserted.

For each cavity cutter in the set of cavity cutters, the length of the cavity cutter measure from the end stop 130 to the curved tip 154 of the blade is the same. That is, when a larger cavity cutter is inserted into the bore, the tip 154 will contact the end of the bore at the same point as for the preceding cavity cutter when the guide sleeve 126 contacts cortical bone, however the central pivot 106 is positioned further out of the bore. Each cavity cutter is arranged to form a larger spherical cavity than that cut by the preceding cavity cutter. Each spherical cavity is tangentially aligned with the end of the bore, rather than being aligned about the same centre point.

Referring now to figure 11, this illustrates in an isometric view the blade end of the instrument when blade 100 has been deployed and has almost reached its finish position. It can be seen that the first and second cutting portions 102,104 extend transverse to the longitudinal axis of the shaft from opposite sides of the shaft. When the first cavity cutter is used to form a spherical cavity, as blade 100 rotates about its central pin 106 separate cavities are cut by each cutting portion 102, 104, with each cavity forming a segment of a sphere. When the blade approaches or reaches its finishing position the cutting edges 156, 158 remove the last portion of bone between each cavity. This will be evident to the surgeon as a sudden reduction in bone resistance to the rotation of the instrument about the axis of shaft 122. Further rotation of blade 100 about central pin 106 is prevented, as discussed above. For each larger cavity cutter used, the two cutting portions progressively and separately enlarge the spherical cavity until the blade approaches or reaches its finishing position and the enlarged portions of the sphere meet.

Referring now to figure 12, this illustrates handle 128 and cutting guide sleeve 126 in a cross sectional view located about a cavity cutter. Handle 128 and cutting guide sleeve 126 are disposed around the shaft 136 and arranged to slide along the shaft. Handle 128 provides a coarse adjustment means for adjusting the length of the cavity guide sleeve 126 extending from the handle 128, and also a fine adjustment means.

Cutting guide sleeve 126 comprises a first portion 164 and a second portion 166 coupled together by a flange 168. The first portion 164 extends from the handle 128 towards the blade end of the instrument. The first portion 164 comprises a narrow sleeve arranged to fit within the bore into the bone. The length of the cutting guide sleeve 126 protruding from the handle 128 can be adjusted once the blade 100 is fully inserted into the bore and curved end 154 is in contact with the end of the bore such that flange 168 rests upon cortical bone surrounding the bore and the handle extends to the end stop 130 forming part of shaft 136. It will be appreciated that in alternative embodiments, the first portion 164 of the cutting guide sleeve may not be arranged to be inserted into the bore, rather it may be the end of the sleeve which rests upon cortical bone surrounding the bore and prevents further movement of the shaft into the bore.

The second portion 166 of the cutting guide sleeve is arranged to be received within the handle 128. The second portion 166 has a screw thread formed in its outer surface. The coarse adjustment means comprises a pair of buttons 170, 172 on either side of the handle 128. Buttons 170, 172 each couple to a ring portion which extends around the second portion 166 of the cutting guide sleeve 126. On the side of each ring remote from the respective button 170, 172 is a portion of a screw thread 174,176 arranged to engage the screw thread on the cutter guide sleeve 126. Each button 170, 172 is biased outwardly by springs 178, 180 such that in the normally biased positions the screw thread portions 174, 176 engage the screw thread on the cutter guide sleeve 126. This prevents the cutting guide sleeve 126 from sliding freely within handle 128. However, the axial position of the guide sleeve 126 relative to the handle 128 can be adjusted by rotating the guide sleeve 126 relative to the handle 128. Applying inwards pressure to both buttons 170, 172 releases screw thread portions 174, 176 from their respective engagement with the screw thread of the guide sleeve 126 such that the guide sleeve 126 can slide freely through the handle 128 to quickly adjust the length of the guide sleeve when the first cutting guide is inserted into the bore.

As noted above, when the screw thread portions 174, 176 engage the screw thread on the second portion 166 of the cutting guide sleeve 126 the cutting guide sleeve can rotate within the handle. This allows for fine adjustment of the cutting guide sleeve position as the screw thread portions 174, 176 travel along the longer screw thread formed on the cutting guide sleeve. This fine adjustment is controlled by a pair of similar buttons 182, 184. Buttons 182, 184 couple to a pair of rings passing around the cutting guide sleeve 126 and again are biased outwardly by springs 186, 188. On the side of each ring remote from the respective button 172, 174 is a lug 190, 192 facing towards the cutting guide sleeve 126 arranged a corresponding groove 194 (not visible in figure 12) extending axially along the surface of the second portion 166 of the cutter guide sleeve 126. The grooves 194 are arranged in pairs on opposite sides of the cutting guide sleeve 126. For instance, there may be four such grooves spaced apart around the circumference of the cutting guide sleeve 126. Applying inwards pressure to both buttons 182, 184 releases lugs 190, 192 from their respective engagement with grooves 194 such that the guide sleeve 126 can freely rotate within the handle 128 to finely adjust the length of the guide sleeve 126 extending from the handle 128.

As discussed above, adjustment of the length of the guide sleeve 126 extending from the handle 128 only needs to be done the first time a cavity cutter is inserted into the bore. As incrementally larger cavity cutters are used the cutting guide sleeve 126 and handle 128 do not require further adjustment. As buttons 170, 172, 182, 184 are outwardly biased, screw thread portions 174, 176 and lugs 190, 192 remain engaged in their respective screw threads and grooves. Consequently, the cutting guide sleeve 126 cannot slide or rotate within the handle 128 preventing inadvertent change in the length of the cutting guide sleeve extending from the handle.

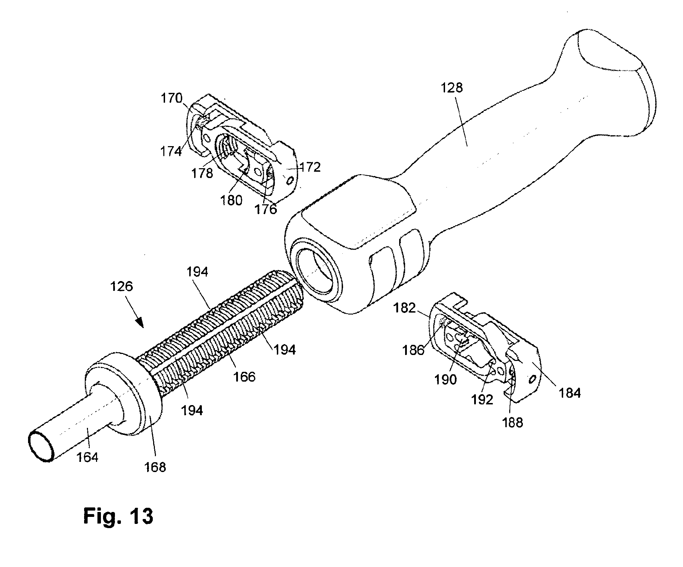

Referring to figure 13, this illustrates the cutting guide sleeve 126 and handle 128 in an exploded view to clarify the form of the coarse and fine adjustment means. It will be appreciated that as an alternative to springs, resilient bands may be used to provide the necessary outwards bias for the buttons, each resilient band extending around both rings for a pair of buttons such that inwards pressure on the buttons causes the rings to slide apart stretching the resilient band.

It will be readily apparent to the skilled person that the design of the handle and cutting guide sleeve may vary, and in particular the adjustment means may vary. The only requirement is for some means of extending the guide sleeve from the handle. Preferably, locking means will be provided to reduce the risk of inadvertent change in the length of the cutting guide sleeve extending from the handle.

Although certain embodiments of the present invention which have been described in detail above relate to forming a cavity in the head of a femur, it will be readily apparent from the teaching herein that the present invention may be applied to form cavities in any bone. Furthermore, it will be readily apparent that certain aspects of the instrument may be altered according to the bone in which a cavity is to be formed. For instance, when used to form cavities in bones smaller than a femur, a smaller blade may be necessary. Typically, instruments in accordance with embodiments of the present invention may be used to form cavities up to 30 mm in diameter.

The blade design may also be altered to adjust the ease of cleaning by allowing access to the pivot points and all cutting surfaces during cleaning. For instance, the coupling of the blade to the shaft may be designed to be partially or fully dismantled. Additionally, in order to cut harder bone notches may be incorporated into the cutting edges 156, 158 to form a serrated edge that assists in detaching chips of harder bone.

Embodiments of the present invention described above primarily relate to forming a cavity in a bone, particularly the head of a femur, for removing necrotic bone tissue for the treatment of avascular necrosis. However, the present invention may be generally applied to forming cavities in any bone in order to remove any form of bone tissue. If will be appreciated that for removing other types of bone tissue the blade design may vary, in particular according to the density of the bone tissue. Furthermore, the dimensions of the instrument may be varied according to the size of the bone in which the cavity is to be formed.

Other modifications to and applications of the present invention will be readily apparent to the appropriately skilled person from the teaching herein, without departing from the scope of the appended claims.

## Claims

1. An instrument (120) for forming a cavity within a bone, the instrument (120) comprising:
a shaft (122);
a blade (100);
a push rod (124) extending within the shaft (122) and coupled to the blade (100), the push rod (120) being arranged to slide within the shaft (122) to cause the blade (100) to rotate about its pivot (106);
a depth stop (126) coupled to the shaft (122) arranged such that when the shaft (122) is inserted into a bore extending into a bone the depth stop (126) is arranged to engage bone surrounding the bore to limit movement of the shaft (122) into the bore; and
a spring (140) coupling the push rod (124) to the shaft (122);
wherein the spring (140) is arranged to resist axial movement of the push rod (124) within the shaft (122) towards the first end of the shaft (122), the instrument (120) being arranged such that when the depth stop (126) engages bone surrounding a bore, axial force exceeding the spring resistance applied to the push rod (124) causes the push rod (124) to slide within the shaft (122), **characterized in that** the blade (100) has two cutting portions (102, 104) pivotally mounted on the shaft (122) towards a first end of the shaft (122) such that the two cutting portions (102, 104) are arranged on opposite sides of the pivot (106).

2. An instrument (120) according to claim 1, wherein the depth stop (126) comprises a guide sleeve (126) extending around the shaft (122) and coupled to the shaft (122).

3. An instrument (120) according to claim 2, further comprising a handle (128) disposed about the shaft (122) and arranged to slide over the shaft (122), the guide sleeve (126) extending over the shaft (127) from the handle (128) such that the shaft (122) can rotate within the handle (128) and the guide sleeve (126).

4. An instrument (120) according to claim 3, wherein the guide sleeve (126) is coupled to the handle (128) such that the length of the cutting guide sleeve (126) extending from the handle (128) is adjustable to adjust the limit of insertion of the shaft (122) into a bore.

5. An instrument (120) according to any one of claims claim 2 to 4, wherein the shaft (127) comprises an end stop (130) positioned towards a second end of the shaft (122), the end stop (130) being arranged to prevent the guide sleeve (126) from passing over the second end of the shaft (122).

6. An instrument (120) according to claim 5, wherein the push rod (124) extends beyond the end stop (130) and terminates at a coupler (132) arranged to connect the instrument (120) to a rotary drive for rotating the push rod (124), shaft (122) and blade (100).

7. An instrument (120) according to claim 6, wherein the spring (140) extends between the coupler (132) and the end stop (130), the spring (140) being arranged to resist an axial force applied to the push rod (124) such that absent of any axial force applied to the push rod (124) the blade (100) is generally aligned with the longitudinal axis of the shaft (122).

8. An instrument (120) according to any one of claims 5 to 7, further comprising a flange (138) coupled to the portion of the push rod (124) extending from the shaft (122), the spring (140) being coupled between the flange (138) and the end stop (130), wherein the spring (140) has a normally biased extended configuration in which the flange (138) is spaced apart from the end stop (130) and a compressed configuration, sliding of the push rod (124) within the shaft (122) being limited by the difference in length of the spring (140) between the extended configuration and the compressed configuration.

9. An instrument (120) according to claim 1, wherein the axis of the pivot (106) about which the blade (100) rotates is perpendicular to the longitudinal axis of the shaft (122), the blade (100) being arranged to rotate about its pivot (106) on the shaft (122) between a first position in which the cutting portions (102, 104) do not extend transversely beyond the side walls of the shaft (122) and a second position in which the first and second cutting portions (102, 104) extend from the shaft (122) transverse to the longitudinal axis of the shaft (122).

10. An instrument (120) according to claim 9, wherein the cutting edge (158) of the first cutting portion (104) is rotationally offset about the pivot (106) of the blade (100) on the shaft (122) from a position diametrically opposite the cutting edge (156) of the second cutting portion (102) in the direction of rotation of the blade (100) between the first and the second positions.

## Patentansprüche

1. Instrument (120) zur Bildung einer Kavität in einem Knochen, wobei das Instrument (120) aufweist:
einen Schaft (122);
eine Klinge (100);
eine Schubstange (124), die sich in dem Schaft (122) erstreckt und mit der Klinge (100) gekoppelt ist, wobei die Schubstange (120) gestaltet ist, um in dem Schaft (122) zu gleiten und zu bewirken, dass sich die Klinge (100) um ihren Drehzapfen (106) dreht;
einen Tiefenanschlag (126), der mit dem Schaft (122) gekoppelt und so gestaltet ist, dass, wenn der Schaft (122) in eine Bohrung eingesetzt ist, die sich in einen Knochen erstreckt, er mit Knochen in Eingriff tritt, der die Bohrung umgibt, um eine Bewegung des Schaftes (122) in die Bohrung zu begrenzen; und
eine Feder (140), die die Schubstange (124) mit dem Schaft (122) koppelt;
wobei die Feder (140) gestaltet ist, um einer axialen Bewegung der Schubstange (124) in dem Schaft (122) in Richtung des ersten Endes des Schaftes (122) zu widerstehen, das Instrument (120) so gestaltet ist, dass, wenn der Tiefenanschlag (126) mit eine Bohrung umgebenden Knochen in Eingriff tritt, eine Axialkraft, die den auf die Schubstange (124) ausgeübten Federwiderstand überschreitet, bewirkt, dass die Schubstange (124) in dem Schaft (122) gleitet, **dadurch gekennzeichnet, dass** die Klinge (100) zwei Schneidteile (102,104) aufweist, die an dem Schaft (122) in Richtung zu einem ersten Ende des Schaftes (122) drehbar montiert sind, sodass die zwei Schneidteile (102,104) auf gegenüberliegenden Seiten des Drehzapfens (106) angeordnet sind.

2. Instrument (120) nach Anspruch 1, wobei der Tiefenanschlag (126) eine Führungsbuchse (126) aufweist, die sich um den Schaft (122) erstreckt und mit dem Schaft (122) gekoppelt ist.

3. Instrument (120) nach Anspruch 2, ferner umfassend einen Griff (128), der um den Schaft (122) angeordnet und gestaltet ist, um über den Schaft (122) zu gleiten, wobei sich die Führungsbuchse (126) über dem Schaft (127) von dem Griff (128) erstreckt, sodass sich der Schaft (122) in dem Griff (128) und der Führungsbuchse (126) drehen kann.

4. Instrument (120) nach Anspruch 3, wobei die Führungsbuchse (126) mit dem Griff (128) so gekoppelt ist, dass die Länge der Schneidführungsbuchse (126), die sich von dem Griff (128) erstreckt, einstellbar ist, um die Grenze des Einfuhrens des Schaftes (122) in eine Bohrung einzustellen.

5. Instrument (120) nach einem der Ansprüche 2 bis 4, wobei der Schaft (127) einen Endanschlag (130) aufweist, der in Richtung eines zweiten Endes des Schaftes (122) positioniert ist und gestaltet ist, um zu verhindern, dass die Führungsbuchse (126) über das zweite Ende des Schaftes (122) tritt.

6. Instrument (120) nach Anspruch 5, wobei sich die Schubstange (124) über den Endanschlag (130) hinaus erstreckt und an einem Koppler (132) endet, der gestaltet ist, um das Instrument (120) mit einem Drehantrieb zum Drehen der Schubstange (124), des Schaftes (122) und der Klinge (100) zu verbinden.

7. Instrument (120) nach Anspruch 6, wobei sich die Feder (140) zwischen dem Koppler (132) und dem Endanschlag (130) erstreckt, wobei die Feder (140) gestaltet ist, um einer auf die Schubstange (124) ausgeübten Axialkraft zu widerstehen, sodass bei Fehlen einer auf die Schubstange (124) ausgeübten Axialkraft die Klinge (100) allgemein mit der Längsachse des Schaftes (122) ausgerichtet ist.

8. Instrument (120) nach einem der Ansprüche 5 bis 7, ferner umfassend einen Flansch (138), der mit dem Teil der Schubstange (124) gekoppelt ist, der sich aus dem Schaft (122) erstreckt, wobei die Feder (140) zwischen dem Flansch (138) und dem Endanschlag (130) angeschlossen ist, wobei die Feder (140) eine normalerweise vorgespannte ausgefahrene Konfiguration, in der der Flansch (138) von dem Endanschlag (130) durch einen Abstand getrennt ist, und eine komprimierte Konfiguration aufweist, wobei ein Gleiten der Schubstange (124) in dem Schaft (122) durch die Differenz der Länge der Feder (140) zwischen der ausgefahrenen Konfiguration und der komprimierten Konfiguration begrenzt wird.

9. Instrument (120) nach Anspruch 1, wobei die Achse des Drehzapfens (106), um die sich die Klinge dreht, senkrecht zur Längsachse des Schaftes (122) ist, wobei die Klinge (100) gestaltet ist, um ihren Drehzapfen (106) an dem Schaft (122) zwischen einer ersten Position, in der sich die Schneidteile (102, 104) nicht quer über die Seitenwände des Schaftes (122) hinaus erstrecken, und einer zweiten Position zu drehen, in der sich die ersten und zweiten Schneidteile (102, 104) von dem Schaft (122) quer zur Längsachse des Schaftes (122) erstrecken.

10. Instrument (120) nach Anspruch 9, wobei die Schneidkante (158) des ersten Schneidteils (104) um den Drehzapfen (106) der Klinge (100) an dem Schaft (122) von einer Position diametral gegenüber der Schneidkante (156) des zweiten Schneidteils (102) in der Richtung der Drehung der Klinge (100) zwischen den ersten und zweiten Positionen drehversetzt ist.

## Revendications

1. Instrument pour former une cavité dans un os, l'instrument (120) comprenant :
un arbre (122) ;
une lame (100) ;
une tige de poussée (124) s'étendant dans l'arbre (122) et couplée à la lame (100), la tige de poussée (120) étant agencée pour coulisser dans l'arbre (122) pour amener la lame (100) à tourner autour de son pivot (106) ;
une butée d'arrêt de profondeur (126) couplée à l'arbre (122), agencée de telle sorte que lorsque l'arbre (122) est inséré dans un perçage s'étendant dans un os, la butée d'arrêt de profondeur (126) est agencée pour venir en prise avec l'os entourant le perçage pour limiter le déplacement de l'arbre (122) dans l'os ; et
un ressort (140) couplant la tige de poussée (124) à l'arbre (122) ;
où le ressort (140) est agencé pour résister à un mouvement axial de la tige de poussée (124) dans l'arbre (122) vers la première extrémité de l'arbre (122), l'instrument (120) étant agencé de façon que lorsque la butée d'arrêt de profondeur (126) vient en prise avec l'os entourant un perçage, une force axiale dépassant la résistance du ressort, appliquée à la tige de poussée (124) amène la tige de poussée (124) à coulisser dans l'arbre (122),
**caractérisé en ce que** la lame (100) possède deux portions coupantes (102, 104) montées d'une manière pivotante sur l'arbre (122) vers une première extrémité de l'arbre (122) de sorte que les deux portions de coupe (102, 104) sont agencées sur des côtés opposés du pivot (106).

2. Instrument (120) selon la revendication 1, dans lequel la butée d'arrêt de profondeur (126) comprend un manchon de guidage (126) s'étendant autour de l'arbre (122) et couplé à l'arbre (122).

3. Instrument (120) selon la revendication 2, comprenant en outre une poignée (128) disposée autour de l'arbre (122) et agencé pour coulisser sur l'arbre (122), le manchon de guidage (126) s'étendant sur l'arbre (127) depuis la poignée (128) de telle sorte que l'arbre (122) peut tourner dans le poignée (128) et le manchon de guidage (126).

4. Instrument (120) selon la revendication 3, dans lequel le manchon de guidage (126) est couplé à la poignée (128) de telle sorte que la longueur du manchon de guidage de coupe (126) s'étendant depuis la poignée (128) est ajustable afin d'ajuster la limite d'insertion de l'arbre (122) dans un perçage.

5. Instrument (120) selon l'une quelconque des revendications 2 à 4, dans lequel l'arbre (127) comprend une butée d'arrêt d'extrémité (130) positionnée vers une seconde extrémité de l'arbre (122), la butée d'arrêt d'extrémité (130) étant agencée pour empêcher que le manchon de guidage passe sur la seconde extrémité de l'arbre (122).

6. Instrument (120) selon la revendication 5, dans lequel la tige de poussée (124) s'étend au-delà de la butée d'arrêt d'extrémité (130) et se termine à un coupleur (132) agencé pour connecter l'instrument (120) à un entraînement rotatif pour faire tourner la tige de poussée (124), l'arbre (122) et la lame (100).

7. Instrument (120) selon la revendication 6, dans lequel le ressort (140) s'étend entre le coupleur (132) et la butée d'arrêt d'extrémité (130), le ressort (140) étant agencé pour résister à une force axiale appliquée à la tige de poussée (124) de telle sorte que, en l'absence de toute force axiale appliquée à la tige de poussée (124), la lame (100) est généralement alignée avec l'axe longitudinal de l'arbre (122).

8. Instrument (120) selon l'une quelconque des revendications 5 à 7, comprenant en outre une bride (138) couplée à la portion de la tige de poussée (124) s'étendant de l'arbre (122), le ressort (140) étant couplé entre la bride (138) et la butée d'arrêt d'extrémité (130), où le ressort (140) a une configuration étendue normalement sollicitée dans laquelle la bride (138) est espacée de la butée d'arrêt d'extrémité (130) et une configuration compressée, le coulissement de la tige de poussée (124) dans l'arbre (122) étant limité par la différence en longueur du ressort (140) entre la configuration étendue et la configuration compressée.

9. Instrument (120) selon la revendication 1, dans lequel l'axe de pivotement (106) autour duquel la lame (100) tourne est perpendiculaire à l'axe longitudinal de l'arbre (122), la lame (100) étant agencée pour tourner autour de son pivot (106) sur l'arbre (122) entre une première position dans laquelle les portions de coupe (102, 104) ne s'étendent pas transversalement au-delà des parois latérales de l'arbre (122) et une seconde position dans laquelle les première et deuxième portions de coupe (102, 104) s'étendent de l'arbre (122) transversal à l'axe longitudinal de l'arbre (122).

10. Instrument (120) selon la revendication 9, dans lequel le bord de coupe (158) de la première portion de coupe (104) est décalé en rotation autour du pivot (106) de la lame (100) sur l'arbre (122) d'une position diamétralement opposée au bord de coupe (156) de la deuxième portion de coupe (102) dans la direction de rotation de la lame (100) entre les première et deuxième positions.
